(19) Europäisches Patentamt
European Patent Office
Office européen des brevets

(11) Publication number: 0 000 804
B1

(12) # EUROPEAN PATENT SPECIFICATION

(45) Date of publication of patent specification: 07.03.84

(51) Int. Cl.³: C 07 D 498/04 //A61K31/42

(21) Application number: 78300135.7

(22) Date of filing: 10.07.78

(54) **Process for the preparation of ethers of clavulanic acid.**

(30) Priority: 04.08.77 GB 3269977

(43) Date of publication of application:
21.02.79 Bulletin 79/4

(45) Publication of the grant of the patent:
07.03.84 Bulletin 84/10

(84) Designated Contracting States:
BE CH DE FR GB NL SE

(56) References cited:
BE - A - 847 045
FR - A - 2 289 188
FR - A - 2 335 512

(73) Proprietor: BEECHAM GROUP PLC
Beecham House Great West Road
Brentford Middlesex (GB)

(72) Inventor: Gasson, Brian Charles
9, Clarence Walk
Redhill Surrey (GB)

(74) Representative: Hesketh, Alan, Dr. et al,
European Patent Attorney Beecham
Pharmaceuticals Great Burgh Yew Tree Bottom
Road
Epsom, Surrey, KT18 5XQ (GB)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 000 804 B1

# Process for the preparation of ethers of clavulanic acid

The present invention relates to a process for the preparation of certain ethers of clavulanic acid.

Ethers of clavulanic acid are disclosed in Belgian Patent No: 847045. It has been found that ethyl and propyl ethers of clavulanic acid and derivatives thereof can conveniently be prepared via the vinyl and propenyl ethers of clavulanic acid.

Accordingly, the present invention provides a process for the preparation of the compounds of the formula (I):

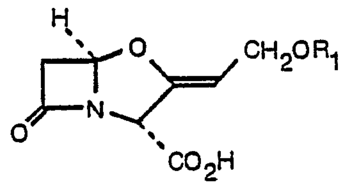

(I)

and salts and esters thereof wherein $R_1$ is a $CH_2CH_3$ or $CH_2CH_2CH_3$ group, which process comprises the reduction of an ester of a compound of the formula (II):

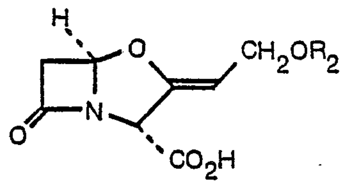

(II)

wherein $R_2$ is a $CH{=}CH_2$ or $CH{=}CH{-}CH_3$ group and thereafter or simultaneously if desired converting the thus-formed ester of the compound of the formula (I) to the corresponding acid or a salt thereof.

Most suitably, the reaction is performed on an ester of the compound of the formula (II) wherein $R_2$ is a $CH{=}CH_2$ group.

The reduction reaction is effected by hydrogenation in the presence of a catalyst such as a transition metal catalyst, such as platinum oxide, palladium or the like. An approximately atmospheric pressure of hydrogen is generally most convenient for laboratory use, but sub-atmospheric pressures may also be employed, so long as extreme conditions are avoided.

If a compound of the formula (I) *per se* or a salt thereof is required, this may be obtained by de-esterifying a suitable ester of the compound, for example, by the hydrolysis of a methoxymethyl or like ester or by the hydrogenolysis of a benzyl, p-methoxybenzyl or like ester optionally in the presence of a base, for example, as described in Belgian Patent No: 847045.

If desired, the hydrogenation of the vinyl or propenyl group and hydrogenolysis of the ester may occur at the same time, by selecting a suitable catalyst and solvent, for example, by using a tetrahydrofuran — containing solvent system and by using a palladium or mixed platinum oxide/palladium catalyst.

Preferably, $R_2$ is a $CH{=}CH_2$ group.

Suitable esters of the compounds of the formula (II) included those wherein the esterifying moiety is a group $A^1$ or $CHA^2A^3$ wherein $A^1$ is an alkyl group of 1—8 carbon atoms optionally substituted by halogen or by a group of the formula $OA^4$, $OCOA^4$, $SA^4$, $SO_2A^4$ wherein $A^4$ is a hydrocarbon group of up to 6 carbon atoms; $A^2$ is a hydrogen atom, an alkyl group of up to 4 carbon atoms or a phenyl group optionally substituted by halogen or by a group $A^5$ or $OA^5$ where $A^5$ is an alkyl group of up to 6 carbon atoms; and $A^3$ is a phenyl group optionally substituted by halogen or nitro or by a group $A^6$ or $OA^6$ where $A^6$ is an alkyl group.

Preferably, the ester of the compound of the formula (II) is such that the corresponding ester of the compound of the formula (I) is a readily hydrolysable or hydrogenolysable ester. Suitable esters of this type include the methoxymethyl, ethoxymethyl, benzyl, methoxybenzyl, nitrobenzyl and the like esters.

A particularly preferred ester of the compounds of the formula (II) is the p-nitrobenzyl ester.

Suitable salts of the compounds of the formula (II) include alkali metal, alkaline earth metal and ammonium and substituted ammonium salts.

Preferably, the salt is a pharmaceutically acceptable salt.

The compounds of formula (II) possess useful $\beta$-lactamase inhibitory properties which allow them to be utilised in antibacterial compositions.

The compositions may be utilised as described in the aforementioned patent specification and may advantageously contain a penicillin or cephalosporin.

The preparation of the compounds of the formula (II) and salts and esters thereof comprise the reaction of an ester of clavulanic acid with a compound of the formula (III) or (IV):

$$R_3 - O - CH = CH_2 \qquad (III)$$

$$R_3 - O - CH = CH - CH_3 \qquad (IV)$$

wherein $R_3$ is an alkyl group of up to 6 carbon atoms, in the presence of mercuric ions as catalyst, and thereafter, if desired, de-esterifying the resulting ester of the compound of the formula (II).

Most suitably, $R_3$ in formulae (III) and (IV) is an ethyl group.

Most suitably, the catalyst is mercuric acetate or mercuric trifluoroacetate.

The reaction may take place in an inert organic solvent, or if a large excess of the compound of the formula (III) or (IV) is used, this may act as solvent.

The reaction may be effected at a non-extreme temperature such as about −10 to 60°C, for example from about 0 to 30°C. Low temperatures in this range are preferred when using a compound of the formula (IV).

Compounds of the formula (II) and salts thereof may be formed by cleavage of the corresponding p-nitrobenzyl ester. This cleavage may be effected using a reducing agent which reduces the nitro group to an amino group, for example, as described in Dutch Patent Application No: 7702027. An example of a suitable reducing agent is iron powder/ammonium chloride solution.

The following Examples 2, 5 and 6 illustrate this invention:

Example 1
Benzyl 9-O-vinylclavulanate

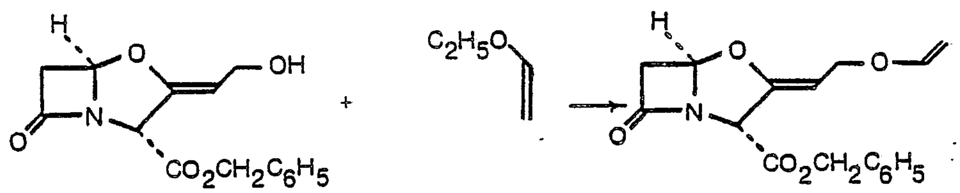

Mercuric acetate (50 mg) was added to a solution of benzyl clavulanate (1 g) in ethyl vinyl ether (10 ml). The solution was refluxed for 6 hours, then allowed to stand at room temperature for 72 hours. The solvent was removed under vacuum and the product purified by column chromatography (Kieselgel G, cyclohexane:ethyl acetate 3:1). Yield 0.676 g. I.r:$\nu_{max}$ (film) 1805, 1750, 1700, 1638, 1620 $cm^{-1}$. N.m.r.: $\delta$ ($CDCl_3$) 3.00 (1H, d, $J$ 17Hz), 3.43 (1H, dd, $J$ 3Hz and 17Hz), 3.95 (1H, dd, $J$ 7Hz and 3Hz), 4.13 (1H, dd, $J$ 3 and 14Hz), 4.26 (2H, d, $J$ 7Hz), 4.81 (1H, broad t, $J$ 7Hz), 5.05 (1H, s), 5.13 (2H, s), 5.64 (1H, d, $J$ 3Hz), 6.33 (1H, dd, $J$ 14 and 7Hz), 7.27 (5H, s)

Example 2
Benzyl 9-O-ethylclavulanate

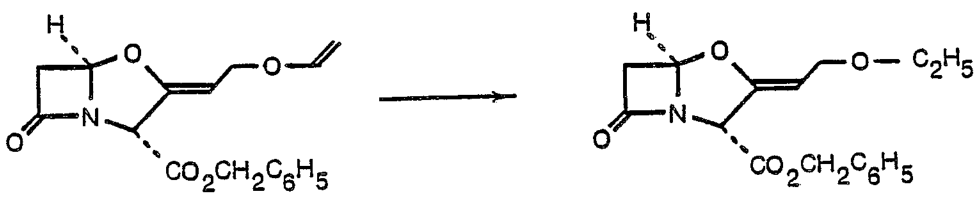

A solution of benzyl 9-O-vinylclavulanate (81 mg) in ethyl acetate (1.0 ml) was hydrogenated over Adams catalyst (10 mg) for 2 hours at room temperature and pressure. The solution was filtered and the filtrate evaporated, and the product separated from starting material by column chromatography (Kieselgel, cyclohexane:ethyl acetate 3:1). Yield 54.8 mg.
I.r$\nu_{max}$ (film) 1805, 1750, 1700 $cm^{-1}$. N.m.r. $\delta$ ($CDCl_3$) 1.16 (3H, t, $J$ 7Hz), 2.98 (1H, d, $J$ 17Hz), 3.37 (2H, q, $J$ 7Hz), 3.41 (1H, dd, $J$ 3 and 17Hz), 4.00 (2H, d, $J$ 7Hz), 4.79 (1H, t, $J$ 7Hz), 5.04 (1H, s), 5.14 (2H, s) 5.62 (1H, d, $J$ 3Hz), 7.29 (5H, s).

Example 3
Methoxymethyl 9-O-vinylclavulanate

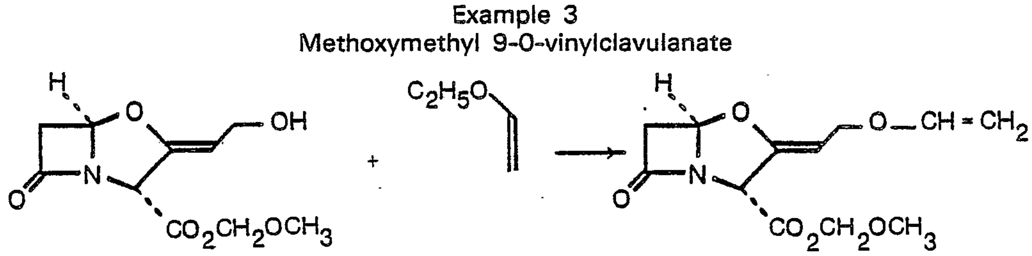

A mixture of methoxymethyl clavulanate (972 mg), ethyl vinyl ether (10 ml) and mercuric acetate (50 mg) was heated at reflux, with stirring, for $3\frac{1}{2}$ hours. The mixture was allowed to stand at room temperature overnight, a further portion of methyl vinyl ether (3 ml) was added, and the mixture was refluxed for a further hour. The solvent was removed under vacuum, and the product was isolated using

column chromatography (Kieselgel 60, cyclohexane:ethyl acetate 3:1) Yield 550 mg as a pale yellow oil.
I.r:$\nu_{max}$ (film) 1810, 1755, 1700, 1635, 1620 $cm^{-1}$. N.m.r. $\delta$ ($CDCl_3$) 3.05 (1H, d, $J$ 17Hz), 3.44 (3H, s), 3.48 (1H, dd, $J$ 3 and 17Hz), 4.97 (1H, dd, $J$ 2 and 7Hz), 4.16 (1H, dd, $J$ 2 and 14hz), 4.31 (2H, d, $J$ 7Hz), 4.90 (1H, broad t, $J$ 7Hz), 5.06 (1H, broad s), 5.22 (1H, d, $J$ 5Hz), 5.33 (1H, d, $J$ 5Hz), 5.68 (1H, d, $J$ 3Hz), 6.36 (1H, dd, $J$ 7 and 14Hz).

## Example 4
### Benzyl 9-O-propenylclavulanate

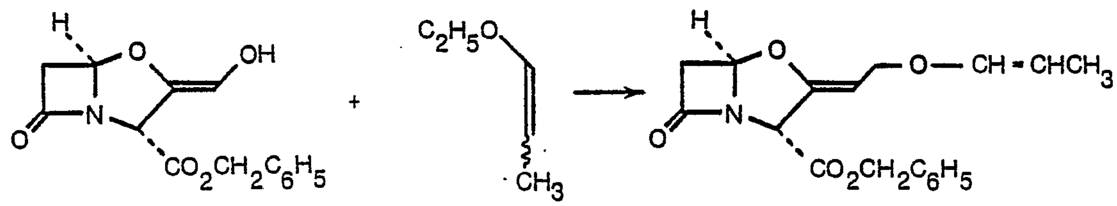

A stirred solution of benzyl clavulanate (578 mg) in ethyl propenyl ether (5 ml) was cooled to 0°C and treated with mercuric trifluoroacetate (20 mg). After 2 hours at 0°C, the mixture was allowed to warm to room temperature for 16 hours. The solvent was removed under reduced pressure and the product was isolated by column chromatography (Kieselgel 60, cyclohexane:ethyl acetate 4:1) Yield 246 mg as a pale yellow oil.
I.r:$\nu_{max}$ (film) 1800, 1750, 1695, 1670 $cm^{-1}$. N.m.r.: $\delta$ ($CDCl_3$) 1.45 – 1.65 (3H, m), 2.99 (1H, d, $J$ 17Hz), 3.44 (1H, dd, $J$ 3 and 17Hz), 4.1 – 4.5 and 4.6 – 4.95 (4H, m), 5.05 (1H, s), 5.13 (2H, s), 5.62 (1H, d, $J$ 3Hz), 5.8 – 6.2 (1H, m), 7.28 (5H, s).

## Example 5
### Benzyl 9-O-propyl clavulanate

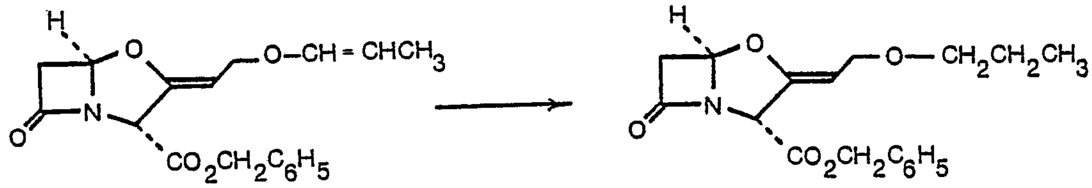

A solution of benzyl 9-O-propenylclavulanate (42.2 mg) in ethyl acetate (3 ml) was hydrogenated over Adams catalyst (10 mg) for 3 hours at room temperature and pressure. The solution was filtered and evaporated to yield the title compound (40.9 mg).
1 spot by t.l.c. (cyclohexane:ethyl acetate 3:1)
I.R.$\nu_{max}$ (film) 1805, 1755 and 1700 $cm^{-1}$. N.m.r. ($CDCl_3$ 0.88 (3H, t, $J$ 7Hz) 1.55 (2H, sextet $J$ 7Hz). 2.99 (1H, d, $J$ 17Hz), 3.27 (2H, t, $J$ 7Hz), 3.42 (1H, dd, $J$ 3 and 17Hz), 3.99 (2H, d, $J$ 7Hz), 4.78 (1H, broad t, 7Hz), 5.04 (1H, s), 5.14 (2H, s), 5.62 (1H, d, $J$ 3Hz), 7.78 (5H, s).

## Example 6
### Sodium 9-O-propyl clavulanate

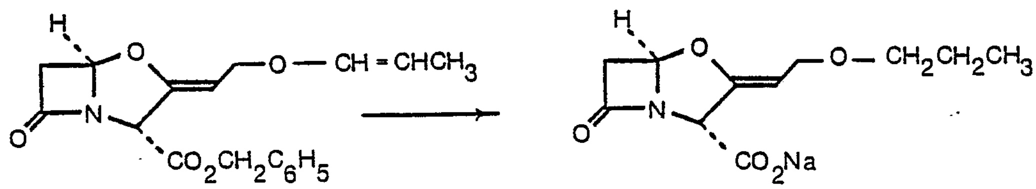

A solution of benzyl 9-O-propenylclavulanate (117 mg) in tetrahydrofuran (4 ml) was hydrogenated over 10% palladium on charcoal (40 mg) at room temperature and pressure for 10 minutes. The solution was filtered through Celite and the residue washed with tetrahydrofuran. The combined filtrates were treated with a solution of sodium bicarbonate (29.9 mg) in distilled water (4 ml). The tetrahydrofuran was then removed on a rotary evaporator. The residual aqueous solution was extracted twice with ethyl acetate and filtered through Celite. The solution was evaporated on a rotary evaporator and the residue dried over phosphorus pentoxide to yield the title compound (77.5 mg).
N.m.r. ($D_2O$) 0.84 (3H, t, $J$ 7Hz), 1.53 (2H, sextet, $J$ 7Hz), 3.05 (1H, d, $J$ 17Hz), 3.41 (2H, t, $J$ 7Hz), 3.54 (1H, dd, $J$ 3 and 17Hz), 4.07 (2H, d, $J$ 7Hz), 4.86 (1H, broad t, $J$ 7Hz), 4.91 (1H, s), 5.69 (1H, d, $J$ 3Hz).
Celite is a registered trade mark.

Example 7

Pivaloyloxymethyl 9-0-vinylclavulanate

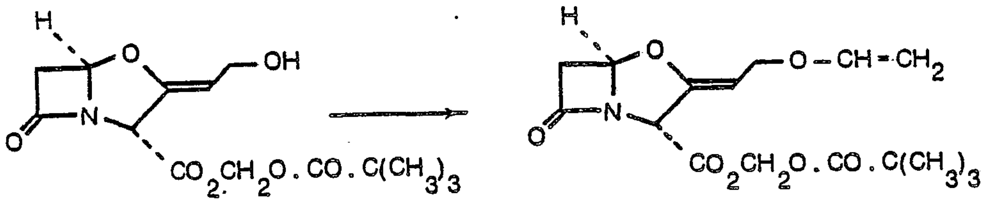

A mixture of pivaloyloxymethyl clavulanate (700 mg of crude material) ethyl vinyl ether (5 ml) and mercuric acetate (50 mg) was refluxed for 6 hours and left to stand at room temperature overnight. The solvent was removed under vacuum and the product isolated by column chromatography (Kieselgel 60, cyclohexane:ethyl acetate 3:1) Yield 19 mg.

I.r:$n_{max}$ (film) 1810, 1760, 1700, 1635, 1620 $cm^{-1}$. N.m.r.: $\delta$ ($CDCl_3$) 1.19 (9H, s), 3.02 (1H, d, $J$ 17Hz), 3.46 (1H, dd, $J$ 3 and 17Hz), 3.96 (1H, dd, $J$ 2.5 and 7Hz), 4.05 – 4.35 (3H, m), 4.84 (1H, broad t, $J$ 7Hz), 5.04 (1H, broad s), 5.66 (1H, d, $J$ 3Hz), 5.71 (1H, d, $J$ 5Hz), 5.78 (1H, d, $J$ 5Hz), 6.35 (1H, dd, $J$ 7 and 14Hz).

Example 8

Methyl 9-0-vinylclavulanate

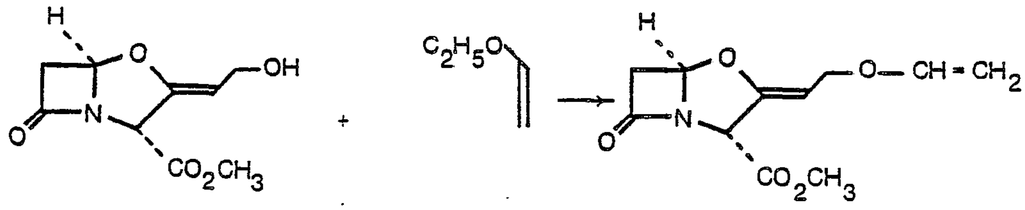

A mixture of methyl clavulanate (426 mg), ethyl vinyl ether (5 ml) and mercuric acetate (25 mg) was heated at reflux for 4 hours. The solvent was removed on a rotary evaporator and the product purified by column chromatography. (Kieselgel, cyclohexane:ethyl acetate 1:1) Yield 168 mg.

I.r.:$\nu_{max}$ (film) 1800, 1750, 1700, 1632, 1618 $cm^{-1}$. N.m.r.: $\delta$ ($CDCl_3$) 3.02 (1H, d, $J$ 17Hz), 3.46 (1H, dd, $J$ 3 and 17Hz), 3.74 (3H, s), 3.97 (1H, dd, $J$ 3 and 7Hz), 4.16 (1H, dd, $J$ 3 and 14Hz), 4.28 (2H, d, $J$ 8Hz), 4.85 (1H, broad t, $J$ 8Hz), 5.04 (1H, s), 5.66 (1H, d, $J$ 3Hz), 6.36 (1H, dd, $J$ 7 and 14Hz).

Analysis: Found C 55.19, H 5.70, N 5.93%

$C_{11}H_{13}NO_5$ requires C 55.23, H 5.48, N 5.86%.

Example 9

*p*-Nitrobenzyl 9-0-vinyl-clavulanate

*p*-Nitrobenzyl clavulanate (3.34 g) was dissolved in dry tetrahydrofuran (30 ml). Ethyl vinyl ether (50 ml) and mercuric acetate (0.5 g) were added to this solution and the resulting mixture was stirred and refluxed (bath temperature 40—50°) with exclusion of moisture for 24 hours. The mixture was filtered and the solvent was evaporated from the filtrate to yield a bright yellow gum. The gum was chromatographed on silica gel (25 g) using ethyl acetate/petroleum ether (b.p. 60—80°). The appropriate fractions, which were recognised using t.l.c., were combined and the solvent was evaporated to give the title compound as a pale yellow gum (270 mg).

$[\alpha]_D^{20} = +31.6°$ ($c$ 1.0, $CHCl_3$). $\nu_{max}$ ($CHCl_3$): 1802, 1750, 1700, 1620, 1615, 1525, 1350 $cm^{-1}$. $\delta$ ($CDCl_3$): 3.07 (1H, d, $J$ 16Hz), 3.51 (1H, dd, $J$ 16 and 2Hz), 3.95—4.15 (2H, m) 4.32 (2H, d, $J$ 8Hz), 4.88 (1H, t, $J$ 8Hz), 5.15 (1H, br.s), 5.28 (2H, s), 5.70 (1H, d, $J$ 2Hz), 6.39 (1H, dd, $J$ 14 and 7Hz), 7.49 (2H, d, $J$ 8.5Hz), 8.22 (2H, d, $J$ 8.5Hz).

Example 10

Lithium 9-0-vinyl-clavulanate

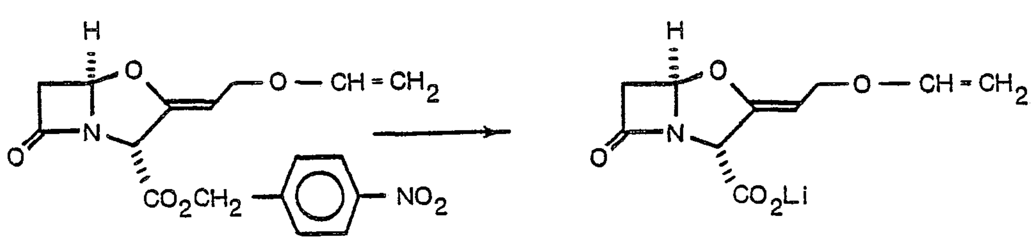

*p*-Nitrobenzyl 9-0-vinyl-clavulanate (190 mg) was dissolved in tetrahydrofuran (6 ml) and to the resulting stirred solution 1*M* ammonium chloride solution (6 ml) and iron powder (0.8 g) were added. The mixture was stirred for 20 minutes and then more iron powder (0.8 g) and 1*M* ammonium chloride solution (0.5 ml) were added. The mixture was stirred for a further 15 minutes and was then diluted with ethyl acetate (100 ml). The resulting mixture was stirred rapidly while hydrogen sulphide was bubbled through it for 5 minutes. The mixture was filtered and the solid was washed well with water. The aqueous layer of the filtrate was saturated with sodium chloride and the mixture was filtered again. The filtrate was treated with 1*N* HCl (3 ml), was shaken, and the layers were separated. The organic layer was dried (sodium sulphate) and filtered. The resulting solution was extracted with 1/15 *M* phosphate buffer (pH 7; 3 × 30 ml). The combined extracts were overlayed with ethyl acetate (50 ml) and were treated with 1*N* HNI (5 ml). The mixture was shaken and the layers were separated. The organic layer was dried (magnesium sulphate) and the solvent was evaporated under reduced pressure. The resulting residue was immediately dissolved in tetrahydrofuran (5 ml). This solution was diluted with water (5 ml) and the pH was adjusted to 7 by dropwise addition of 0.1 *M* lithium hydroxide solution (*ca* 2 ml). The neutralised solution was filtered and the solvent was evaporated from the filtrate under reduced pressure. The resulting residue was stirred with a mixture of acetone (3 ml) and ether (6 ml). The resulting solid was collected by filtration, washed with ether, and dried *in vacuo*.

The title compound was thus obtained as a very pale yellow powder which appeared to be 70—80% pure as judged by t.l.c. and n.m.r.

$\nu_{max}$ (KBr): 1770, 1690, 1610 $cm^{-1}$. $\delta$ ($D_2O$); *inter alia:* 3.02 (1H, d, *J* 17Hz), 3.50 (1H, dd, *J* 17 and 2.5Hz), 4.00—4.25 (2H, m), 5.68 (1H, d, *J* 2.5 Hz), 6.42 (1H, dd, *J* 14 and 7Hz).

Demonstration of $\beta$-Lactamase Inhibitory Activity

The minimum inhibitory concentrations (MIC) of certain compounds prepared above and of ampicillin, alone or in the presence of compounds of the invention, were determined *in vitro* for a range of micro-organisms.

The results are shown in Table 1.

TABLE 1

| Compound of Example No. | Conc. µg/ml | MIC Ampicillin µg/ml | | | |
|---|---|---|---|---|---|
| | | SAR* | KA* | PM* | EC* |
| 8 | 5 | 0.6 | 3.1 | 8 | 8 |
| | 1 | 1.25 | 3.1 | 62.5 | 16 |
| | 0 | 250. | 62.5 | 500 | 250 |
| Compound alone | | 125 | 500 | 250 | 500 |
| 10 | 5 | 0.08 | 3.12 | 2 | 4 |
| | 1 | 0.3 | 3.12 | 8 | 8 |
| | 0 | 1000 | 1000 | >2000 | 2000 |
| Compound alone | | 15.6 | 31.2 | 62.5 | 31.2 |

*SAR : *Staphylococcus aureus* Russell

KA : *Klebsiella aerogenes* E70

PM : *Proteus mirabilis* C889

EC : *E. coli* JT39

**Claims**

1. A process for the preparation of a compound of the formula (I):

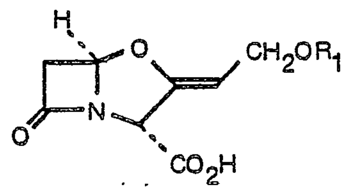

(I)

wherein $R_1$ is a $CH_2CH_3$ or $CH_2CH_2CH_3$ group or a salt or ester thereof, which process comprises the reduction by hydrogenation in the presence of a transition metal catalyst of an ester of a compound of the formula (II):

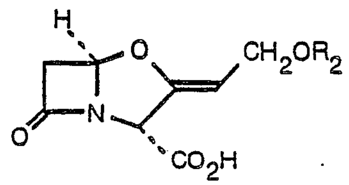

(II)

wherein $R_2$ is a $CH{=}CH_2$ or $CH{=}CH{-}CH_3$ group and thereafter or simultaneously if desired converting the thus-formed ester of the compound of the formula (I) to the corresponding acid or a salt thereof.

2. A process as claimed in claim 1 wherein $R_2$ is a $CH{=}CH_2$ group.

3. A process as claimed in claim 1 wherein the catalyst is platinum oxide or palladium.

4. A process as claimed in any one of claims 1—3 for the preparation of a compound of the formula (I) or a salt thereof wherein the methoxymethyl ester of the compound of the formula (I) is produced and de-esterified by hydrolysis.

5. A process as claimed in any one of claims 1—3 for the preparation of a compound of the formula (I) or a salt thereof wherein the benzyl or p-methoxybenzyl ester of the compound of the formula (I) is produced and de-esterified by hydrogenolysis, optionally in the presence of a base.

6. A process as claimed in any one of claims 1—3 and 5 wherein the hydrogenation of the vinyl or propenyl group and hydrogenolysis of the ester occur at the same time.

**Patentansprüche**

1. Ein Verfahren zur Herstellung einer Verbindung der Formel (I)

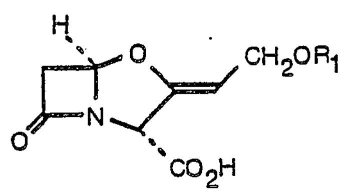

(I)

in der $R_1$ eine $CH_2CH_3$- oder $CH_2CH_2CH_3$-Gruppe ist, deren Salz oder Ester, wobei das Verfahren die Maßnahmen der Reduktion eines Esters einer Verbindung der Formel (II)

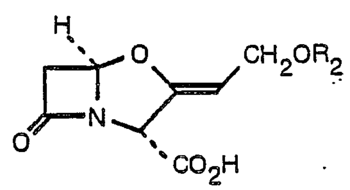

(II)

in der $R_2$ eine $CH{=}CH_2$- oder $CH{=}CH{-}CH_3$-Gruppe ist, durch Hydrierung in Gegenwart eines Übergangsmetallkatalysators und danach oder gleichzeitig- gewünschtenfalls- Umwandlung des derart gebildeten Esters der Verbindung der Formel (I) in die entsprechende Säure oder deren Salz umfaßt.

2. Ein Verfahren wie in Anspruch 1 beansprucht, wobei $R_2$ eine $CH{=}CH_2$-Gruppe ist.

3. Ein Verfahren wie in Anspruch 1 oder 2 beansprucht, wobei der Katalysator Platinoxid oder Palladium ist.

4. Ein Verfahren wie in irgendeinem der Ansprüche 1 bis 3 beansprucht für die Herstellung einer Verbindung der Formel (I) oder deren Salz, wobei der Methoxymethylester der Verbindung der Formel (I) erzeugt und durch Hydrolyse entestert wird.

5. Ein Verfahren wie in irgendeinem der Ansprüche 1 bis 3 beansprucht für die Herstellung einer Verbindung der Formel (I) oder deren Salz, wobei der Benzyl- oder p-Methoxybenzylester der Verbin-

dung der Formel (I) erzeugt und durch Hydro genolyse gegebenenfalls in Gegenwart einer Base, entestert wird.

6. Ein Verfahren wie in irgendeinem der Ansprüche 1 bis 3 und 5 beansprucht, wobei die Hydrierung der Vinyl- oder Propenyl-gruppe und die Hydrogenolyse des Esters gleichzeitig stattfindet.

**Revendications**

1. Procédé pour la préparation d'un composé de formule (I):

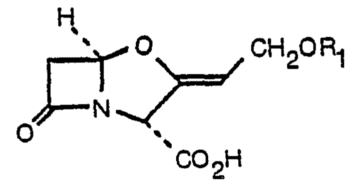

(I)

dans laquelle $R_1$ est un groupe $CH_2CH_3$ ou $CH_2CH_2CH_3$, ou d'un sel ou ester de ce composé, ce procédé consistant à effectuer la réduction, par hydrogénation en présence d'un catalyseur à base de métal de transition, d'un ester d'un composé de formule (II):

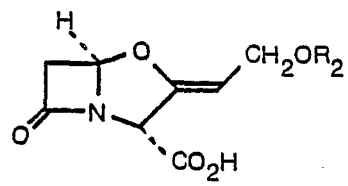

(II)

dans laquelle $R_2$ est un groupe $CH{=}CH_2$ ou $CH{=}CH{-}CH_3$ et, ensuite ou simultanément, si désiré à convertir l'ester ainsi formé du composé de formule (I) en acide correspondant ou en un sel de celui-ci.

2. Procédé suivant la revendication 1, caractérisé en ce que $R_2$ est un groupe $CH{=}CH_2$.

3. Procédé suivant la revendication 1, caractérisé en ce que le catalyseur est de l'oxyde de platine ou du palladium.

4. Procédé suivant l'une quelconque des revendications 1 à 3 pour la préparation d'un composé de formule (I) ou d'un sel de celui-ci, selon lequel l'ester méthoxyméthylique du composé de formule (I) est produit et désestérifié par hydrolyse.

5. Procédé suivant l'une quelconque des revendications 1 à 3 pour la préparation d'un composé de formule (I) ou d'un sel de celui-ci, selon lequel l'ester benzylique ou p-méthoxybenzylique du composé de formule (I) est produit et désestérifié par hydrogénolyse, facultativement en présence d'un base.

6. Procédé suivant l'une quelconque des revendications 1 à 3 et 5, selon lequel l'hydrogénation du groupe vinylique ou propénylique et l'hydrogénolyse de l'ester se produisent en même temps.